# EUROPEAN PATENT SPECIFICATION

(11) **EP 3 801 418 B1**
(45) Date of publication and mention of the grant of the patent: **11.03.2026**
(21) Application number: 19729833.4
(22) Date of filing: 08.05.2019
(51) Int. Cl.: A61F 13/08, A61F 13/06, A61K 47/34, A61L 15/26, A61L 15/40, A61L 15/44, A61K 9/70

(54) **FABRIC FOR GARMENTS AND COMPRESSION, RELATED CONTENITIVE GARMENTS AND METHOD FOR THE PRODUCTION THEREOF**
GEWEBE FÜR KLEIDUNGSSTÜCKE UND KOMPRESSION, VERWANDTE KOMPRESSIONSKLEIDUNGSSTÜCKE UND VERFAHREN ZU IHRER HERSTELLUNG
TISSU POUR VÊTEMENTS ET COMPRESSION, VÊTEMENTS DE CONTENTION ASSOCIÉS ET LEUR PROCÉDÉ DE PRODUCTION

(30) Priority: 08.05.2018 IT 201800005140
(43) Date of publication of application: 14.04.2021
(73) Proprietor: Federico, Giuseppe Leonardo, 8032 Zurigo (CH); Gloria Med Pharma Srl, 22017 Menaggio (CO) (IT)
(72) Inventor: ROSSI, Elvira, 42011 San Tomaso - Bagnolo in Piano (RE) (IT); FEDERICO, Giuseppe Leonardo, 8032 Zurigo (CH)
(74) Representative: Maiello, Helenio Francesco
(86) International application number: PCT/IB2019/053760
(87) International publication number: WO 2019/215624

(56) References cited:
- EP-A1- 1 741 415
- EP-A1- 1 892 324
- EP-A1- 2 027 838
- WO-A2-2006/065854
- IT-A1- 201800 005 140
- US-A1- 2007 207 688
- US-A1- 2014 023 610

## Description

### Technical Field

The present invention finds application in the field of garments and bandages for the aesthetic and/or medical treatment and it particularly relates to a fabric for production of bandages and/or compression garments as well as a compression garment with compression effect and osmotic and draining action made with said fabric.

The invention also relates to a method for manufacturing the above fabric with compressive and healing properties.

### State of the art

Several types of containing clothing are known, such as bands, girdles, trousers, undergarments, waistcoats and the like, composed of more or less elastic fabrics whose main purpose is to model or in any case contain the parts of the body on which they are worn, generally for purely aesthetic purposes.

However, these garments have several drawbacks which make their use inconvenient, both in the short and long term, and which can also cause some physical discomforts. First of all, the garments currently on the market are usually non-breathable and can thus create localized increases in body temperature and accumulations of sweat at the covered areas, with the possibility of skin irritations.

Furthermore, these garments have no beneficial effect on the body but have only an aesthetic function.

In particular, in the context of the treatment of blemishes and cutaneous traumas, such as cellulite, scars, with particular attention to the formation of keloids linked to the abnormal production of scar tissue following the repair processes of skin wounds, or other infectious skin traumas , or consequent to surgical interventions, the therapeutic options currently available are numerous and include both non-invasive therapies based on synthetic polymers to be applied on the lesion, and more aggressive and invasive surgical and pharmacological methods.

Between the topical treatments, silicone-based gels and other synthetic polymers are largely used in the form of gels or foils for the topical treatment of scars. These products can be applied both on closed wounds, in the process of healing, and on hypertrophic scars or keloids already formed, with the aim, in the first case, of guaranteeing optimal healing and, in the second case, of improving the appearance of the injury and a reduction in any associated symptoms.

In particular, the use of silicone materials has proved to be particularly advantageous in that these materials have demonstrated several extremely interesting properties. For example, some of the properties of these materials are represented by their great stability, great resistance to ultraviolet radiation, acids, alkalis, electric charges, thanks to the chemical origin of silicon and the strong bonds that distinguish it. Furthermore, the silicone is insoluble in water, but it is permeable to water vapor, allowing the skin a good transpiration and thus preventing skin lesions linked to the formation of sweat rush, which often occur after the use of occlusive products. Another important feature is constituted by the substantivity of these materials, that is the ability to form a film adherent to the keratin of skin and hair.

Therefore, any substance conveyed with silicone becomes resistant to water and rubbing.

Several studies have evaluated the efficacy of silicone materials in the treatment of scarring (hypertrophic scars and keloids) and have shown the superiority of silicone gel in reducing the size of scars and improving the appearance of lesions.

In a controlled study, conducted on 18 patients with cheloidal scars, a flattening was observed in 64% of the lesions after 6 months of constant use of the dressing; the best results were obtained with smaller lesions.

The use of silicone gels has proved preferable over the use of silicone foils in the treatment of localized lesions, for example, at the level of the ear lobe or of the sternum where the laminae could, on the contrary, be more difficult to apply and/or to be maintained in situ.

By contrast, the laminae can represent a more suitable solution for extensive scars located on flat skin areas (e.g. limbs, abdomen).

The silicone gel has the advantage of being more comfortable and practical and should be applied in a thin layer on the lesion and left to dry for 4-5 minutes.

The laminae are applied by pressure and must be worn for at least 12 hours a day, so they can be removed and washed before being reused the next day.

In both cases, however, the need is felt for a product that allows a more controlled and gradual release of the substances and active ingredients related to the silicone materials, as well as greater ergonomics of the treatment devices and an efficacy also with respect to the different aesthetic blemishes, including blemishes related to panniculopathy and similar diseases.

EP1892324 discloses a sheet material comprising a layer of elastic fabric and a layer of adhesive silicone gel coating for applications on a part of the body in order to reduce skin friction and/or skin lesions during exercise and/or sports practice.

However, the silicones used exhibit non-optimal spreadability, with consequent non-optimal adhesion to the support fabric layer, and poor breathability, which makes it necessary to drill holes in the silicone layer in correspondence with eyelets present in the fabric layer.

### Scope of the invention

The object of the present invention is to overcome the above drawbacks by providing a fabric for making bandages and/or compression garments that is particularly effective and relatively inexpensive.

A particular object is to provide a fabric for the production of bandages and/or compression garments which have features of high elastocompression and biocompatibility.

Still another particular object is to provide a fabric for the manufacture of bandages and/or compression garments which allows to use the synergy between elastocompression and occlusive action of the silicone so as to avoid the need to provide the overlapping of silicone sheets and uncomfortable bandages, to avoid the approximate compression, often harmful if excessive and definitely useless if too bland.

A further object is to provide a fabric for the production of bandages and/or compression garments which aid the lymphatic system to expel the bulky residues of the human body.

A further object is to provide a fabric for the production of bandages and/or compression garments that is made starting from low viscosity silicone materials in order to favour the uniform application thereof on the fabric layer.

A further purpose is to provide a fabric for the manufacture of bandages and/or compression garments that has high breathability or gas permeability.

Yet another particular object is to provide a containing garment that has breathable and draining properties, in order to avoid body overheating and at the same time improving circulation, so as to be comfortable once worn and to produce beneficial effects on the human body.

Still another object is to provide a containing garment that is completely hypoallergenic, non-toxic and does not irritate the skin.

Still another object is to provide a method for producing a fabric for the manufacture of bandages and/or compression garments which allows to apply the silicone material in a simple and uniform manner, even manually.

These objects, as well as others which will become more apparent hereinafter, are achieved by a fabric which, according to claim 1, comprises an elastocompressive layer consisting of yarns having an elastic component and a gas-permeable coating membrane applied to a face of said elastocompressive layer and obtained starting from a mixture of pure medical grade silicone materials, said mixture comprising low viscosity cyclosilicones and one or more silicone materials with healing properties (scar gel).

According to a further aspect of the invention, a garment according to claim 12 is provided, characterized in that it consists of a fabric comprising an elastocompressive layer consisting of yarns having an elastic component and a gas-permeable coating membrane applied to a face of said elastocompressive layer and consisting of a mixture of pure medical grade silicone materials, said mixture comprising low viscosity cyclosilicones and one or more silicone materials with healing properties (scar gel), wherein said membrane is distributed on the side of said fabric designed to be facing, in use, towards the user's skin.

According to a further aspect of the invention there is provided a method for the production of fabrics according to claim 13, which method comprises the steps of providing an elastocompressive layer consisting of yarns having an elastic component, application on one face of said elastocompressive layer of a mixture of pure medical grade silicone materials comprising low viscosity cyclosilicones and one or more silicone materials with scar-healing properties, cross-linking of said mixture to obtain a coating membrane, wherein said application step is carried out by spreading said mixture of silicone materials on said elastocompressive layer.

Advantageous embodiments of the invention are obtained according to the dependent claims.

### Best modes of carrying out the invention

The fabric according to the present invention may be designed in different ways based on the part of the body on which it will be applied.

In a non-limiting manner, the fabric may be used for manufacturing compressive garments, such as trousers or other garments destined to be worn on the legs, for example short pants or the like, contentive girdles, abdominal bands, waistcoats, bras or other undergarments, without particular limitations.

The fabric may also be used to make bandages, tubulars, plaques or even simple pads or plasters for applications using auxiliary retaining or locking bands.

In its most general embodiment, the fabric will consist of a support layer composed of elastic yarns in natural and/or synthetic fibers having elastic-compressive properties and coated with a layer of pure medical grade silicone material.

By way of example, the support layer may be mainly made of synthetic fibers with inserts in natural fibers arranged at the areas designed to come into contact, in use, with the private parts of the user or with other particularly sensitive parts. According to a particular embodiment, the yarns may comprise an internal component in elastic fiber and an external component in natural (for example cotton) and/or synthetic fiber.

The elastic synthetic fibers will preferably be microfibres and in particular a compound of fibers with different elasticity will be used, for example polyamide and polyurethane fibers.

According to a particularly preferred embodiment, the microfibres may be composed of polyamide in a percentage ranging from 70% to 95%, preferably from 75% to 85%, and spandex in a complementary percentage to 100%.

A particularly preferred composition comprises 84% polyamide fibers and 16% polyurethane fibers.

Advantageously, the silicone materials will be medical grade 1, so as to ensure high biocompatibility and not create irritation on the skin.

Moreover, the membrane will also be gas-permeable, to avoid the accumulation of sweat in the areas in contact with the human body, not causing rashes and not heating the muscle.

To this end, the membrane will be obtained by cross-linking a starting mixture of silicone materials comprising vinylpolysiloxane, which will favour the transpiration and consequently the microcirculation, guaranteeing the spontaneous overproduction of elastin and collagen which also allows the aesthetic improvement of the dermis. Preferably, the silicone materials will be silicone gels and may be distributed on natural and/or synthetic fibers either by cold or, preferably, hot spreading.

According to the invention, the silicone material will be a silicone gel formed starting from a mixture of silicone materials comprising one or more elastomeric materials in a percentage ranging from 20% to 40%, a low viscosity silicone material selected from the cyclosilicones in a percentage comprised between 40% and 70% and one or more silicone materials (SCAR GEL) with healing properties in a percentage comprised between 1% and 10%, preferably close to 5%.

The presence of cyclosilicones is essential to reduce viscosity and make the mixture spreadable on fabric but they are almost irrelevant on the subsequent cross-linking phase, as they are volatile, so only few traces of them remain after polymerization. The composition of the biocompatible materials allows to obtain the gradual release effect, since it will be possible to include, during the catalysis of the components, added substances and active ingredients that are gradually released by the plates or by relative garments.

By way of example, the active ingredients may be selected among principles with a lightening effect, such as standardized liquid or powdered vegetable extract, based on liquorice titrated in acid, glycyrrhetinic, water-soluble, standardized both liquid and powdered vegetable extract, based on grapes ursina titrated in arbutin, water-soluble, complex of extracts of cucumber, lemon fruit and sodium citrate in synergistic mixture with a high anti-stain and anti-edema action, water-soluble, with an anti-cellulite effect, such as a plant complex titrated with active ingredients, based on iodized estensin, caffeine , flavonoids, ruscogenin, water-soluble, plant-based ingredient derived from mirica cerifera L. titrated in dihydromiricetin, both liquid and powder, water-soluble, anti-aging effect, as a multifunctional complex based on farnesol, vitageno F and their derivatives, fat-soluble or water-soluble , polyfunctional complex based on adenosine triphosphate, chondroitin sulphate, vegetable protein, water-soluble, poly-functional complex based on beta-glucan, obtained from biotechnologies, water-soluble, polyfunctional complex based on adenosine triphosphate, nicotinamide, milk-soluble proteins, water-soluble, polyfunctional complex based on dioscorea villosa, sterols of soybean wisteria, oleyl alcohol, complex based on dioscorea villosa, sterols of soybean wisteria, oleyl alcohol, vegetable , liposoluble, multi-purpose complex based on beta vulgaris root extract, haberlea rhodopensis foglie, faex, in vegetable glycerine, water-soluble, poly-functional complex of polysaccharides based on extract of tamarindus indica, vegetable, water-soluble, multifunctional complex based on farnesol, panthenol and their derivatives, fat-soluble or water-soluble, vegetable synergistic complex based on milk thistle, peptides, silica, tannins with a firming and anti-stretch mark action, liquid, water-soluble, with a controlled release vehicular effect, such as stable, naturally analogous aqueous nanodispersions, with particles of 50 -150 nm based on plant triglycerides and phosph atidylcholine, for the conveyance of liposoluble active ingredients such as vitamin A and E.

Their particular composition also allows the formulation of cosmetics in the form of gels containing otherwise water-soluble vitamins, such as beads based on microcrystalline cellulose, hydroxypropyl methylcellulose, lactose, sericin, cellulose-based microspheres of different colors with a visualizing effect, lactose or mannitol, all of which loaded with vitamin E either alone or mixed with vitamin A and C.

It is also possible to add the mixture with reconstituted moisturizing factors, such as a synergistic mixture of different amino acids and PCA, water-soluble, concentrated complex based on analine, creatine, glycerin, glycine, magnesium aspartate, natural saccharides, urea, water-soluble

A possible formulation made using Dow Corning silicones is shown in the following table:

| | |
|---|---|
| MDX4-4210 Base | 30.0 % |
| MDX4-4210 Curing | 3.0 % |
| MG 7-9800 part A | 1.0 % |
| MG 7-9800 part B | 1.0 % |
| ST-Cyclomethicone 5-NF | 60.0 % |
| Q7-9120 Silicone | 5% |
| Fluid 12,500 cSt | |
| TI-3021 Silicone Elastomer Blend | (SCAR GEL) |
| TI-7021 Silicone Resin Blend | |

The preferred method for obtaining the crosslinked fabric is the manual spreading of the mixture of silicone elastomers on the fabric item; the coating is carried out manually with a brush with synthetic bristles on the inner side of the item, which wiol be then in contact with the skin.

After spreading, cross-linking is strongly favoured by temperature and humidity. For this reason, ventilated ovens are used as ventilation is necessary to have the same temperature in every part of the item and to have a uniform reticulation.

A preferred cross-linking temperature is 70° C while the cross-linking time changes according to the item, the mass thereof, the yarn texture and with average values ranging between 30 min to a maximum of 2 hours.

Polymerization may also take place at room temperature, although in this case it takes about 48/60 hours to complete the process.

In both cases crosslinking may be considered substantially completed after 72 hours. The fabric thus created has osmotic properties because the silicone material will partially occlude the pores of the skin, favouring the osmotic process and allowing the ion exchange between oxygen and carbon dioxide, attracting water molecules not assimilated to the toxin that cause inflammation on the surface.

The viscous and elastic properties of the silicone gel will not produce irritation on the skin, avoiding abrasions.

At the same time, the membrane will release mineral oils that will come into contact with the skin, favouring semi-permeable osmotic filling, so as to rehydrate the stratum corneum by osmosis and making the skin softer even without the additional use of creams and lotions.

The elastocompressive effect and the osmotic effect will counteract water retention, with beneficial effects also in anti-cellulite treatments, favouring the drainage of lactic acid, improving vascularization and increasing tissue oxygenation levels and subcutaneous drainage of toxins.

Since the fibers are completely covered by the silicone material, they are isolated from contact with the skin and therefore there will be no production of germs or bacteria at the skin, also thanks to the germicidal properties of the membrane.

In this way the garment may also be used in treatments against atopic dermatitis (chronic eczema).

The compression garments made of medical silicone increase the flow of return to the heart and significantly reduce venous insufficiency, that is the discomfort of swelling of the lower limbs, conspicuously limiting the first varicose veins and containing the rapid chronic action of Edematous Fibrosclerotic Panniculopath (EFP).

Thanks to the compressive properties, the garment also performs an anti-inflammatory action, stimulates the spontaneous production of elastin and collagen, for the aesthetic improvement of the dermis, improves the absorption of treating substances, tones muscle mass and reduces post-treatment edema.

The garment will be particularly suitable for localized slimming or water retention treatments, cellulite, vascular disorders, inflammatory conditions, joint pain, muscle pain, joint surmenage, muscle relaxation. inflammatory lesions, bruises and sprains, circulatory disorders , muscle relaxation, traumatic contractures, sports injuries, treatment and prevention of PEF (cellulite) stages, in modeling treatments following liposuction procedures, as it limits the formation of scars and lesions and reduces post-surgical rehabilitation times.

The tissue also helps the lymphatic system to expel the bulky residues of the human body, avoiding the accumulation of tinned liquids from the tissue lymph which is not only painful but often leads to localized and systemic inflammation including PEF.

## Claims

1. A fabric for the production of compression bandages and/or garments comprising:
- an elastocompressive layer consisting of yarns having an elastic component;
- a gas-permeable coating membrane applied to a face of said elastocompressive layer;
wherein said coating membrane is a layer of pure medical grade silicone gel obtained by cross-linking starting from a starting mixture of pure medical grade silicone materials which comprises one or more elastomeric materials, one or more low viscosity silicone materials selected from cyclosilicones and one or more silicone materials with healing properties;
wherein said elastomeric materials are present in said starting mixture in a percentage ranging from 20% to 40% by weight of said mixture and said one or more silicone materials with healing properties are present in said starting mixture in a percentage comprised between 1% and 10% by weight of said mixture;
**characterized in that** said low viscosity silicone materials selected from the cyclosilicones are present in a percentage comprised between 40% and 70% by weight of said mixture.

2. Fabric as claimed in claim 1, **characterized in that** said silicone material is of medical grade 1.

3. Fabric as claimed in any preceding claim, **characterized in that** said one or more silicone materials with healing properties are present in said starting mixture in a percentage close to 5% of the weight of said mixture.

4. Fabric as claimed in any preceding claim, **characterized in that** said mixture of silicone materials comprises vinylpolysiloxane.

5. Fabric as claimed in any preceding claim, **characterized in that** said mixture of silicone materials is added with one or more active principles.

6. Fabric as claimed in claim 5, **characterized in that** said active principles are selected from the group comprising plant extracts, complexes of plant extracts, liposoluble and water-soluble polyfunctional complexes.

7. Fabric as claimed in any preceding claim, **characterized in that** said silicone gel is distributed by hot spreading on a single face of said elastocompressive layer.

8. Fabric as claimed in any preceding claim, **characterized in that** said yarns comprise an internal component in elastic fiber and an external component in natural and/or synthetic fiber.

9. Fabric as claimed in claim 8, **characterized in that** said elastic fibers are selected from the group comprising the microfibres.

10. Fabric as claimed in claim 9, **characterized in that** said microfibres are polyamide and/or polyurethane fibers.

11. Fabric as claimed in claim 10, **characterized in that** said microfibres are composed of polyamide in a percentage ranging between 70% and 95%, preferably between 75% and 85% and spandex in a percentage complementary to 100%.

12. A content garment **characterized in that** it consists of a fabric according to any claim from 1 to 11, wherein said membrane is distributed on the side of said fabric designed to be turned, in use, towards the user's skin.

13. A method for the manufacturing of fabrics according to any claim from 1 to 11 for the production of bandages and/or compression garments, which method comprises the following steps:
a) providing an elastocompression layer consisting of yarns having an elastic component;
b) applying on one face of said elastocompressive layer the mixture of pure medical grade silicone materials;
c) cross-linking of said mixture to obtain the coating membrane;
wherein said applying step b) is carried out by spreading said mixture of silicone materials on said elastocompressive layer.

## Patentansprüche

1. Ein Gewebe zur Herstellung von Kompressionsverbänden und/oder - kleidungsstücken, umfassend:
- eine elastokompressive Schicht aus Garnen mit elastischer Komponente;
- eine gasdurchlässige Beschichtungsmembran, die auf die Oberfläche der elastokompressiven Schicht aufgebracht ist;
wobei es sich bei der Beschichtungsmembran um eine Schicht aus reinem medizinischem Silikongel handelt, die durch Vernetzung aus einer Ausgangsmischung von reinen medizinischen Silikonmaterialien gewonnen wird, welche ein oder mehrere elastomere Materialien, ein oder mehrere niedrigviskose Silikonmaterialien, ausgewählt aus Cyclosilikonen, und ein oder mehrere Silikonmaterialien mit heilenden Eigenschaften umfasst;
wobei die elastomeren Materialien in der Ausgangsmischung in einem Gewichtsanteil von 20 bis 40 % und die ein oder mehreren Silikonmaterialien mit heilenden Eigenschaften in einem Gewichtsanteil von 1 bis 10 % enthalten sind;
**dadurch gekennzeichnet, dass** die genannten niedrigviskosen Silikonmaterialien, ausgewählt aus den Cyclosilikonen, in einem Gewichtsanteil zwischen 40 und 70 % der Mischung vorliegen.

2. Gewebe nach Anspruch 1, **dadurch gekennzeichnet, dass** das Silikonmaterial von medizinischer Qualität ist.

3. Gewebe nach einem vorhergehenden Anspruch, **dadurch gekennzeichnet, dass** ein oder mehrere Silikonmaterialien mit heilenden Eigenschaften in der Ausgangsmischung in einer Konzentration von etwa 5 Gew.-% der Mischung enthalten sind.

4. Gewebe nach einem vorhergehenden Anspruch, **dadurch gekennzeichnet, dass** die Mischung aus Silikonmaterialien Vinylpolysiloxan enthält.

5. Gewebe nach einem vorhergehenden Anspruch, **dadurch gekennzeichnet, dass** der Mischung aus Silikonmaterialien ein oder mehrere Wirkstoffe zugesetzt sind.

6. Gewebe nach Anspruch 5, **dadurch gekennzeichnet, dass** die Wirkstoffe ausgewählt sind aus der Gruppe, die Pflanzenextrakte, Komplexe aus Pflanzenextrakten, fettlösliche und wasserlösliche polyfunktionelle Komplexe umfasst.

7. Gewebe nach einem vorhergehenden Anspruch, **dadurch gekennzeichnet, dass** das Silikongel durch Heißverstreichen auf einer Seite der elastokompressiven Schicht verteilt wird.

8. Gewebe nach einem vorhergehenden Anspruch, **dadurch gekennzeichnet, dass** die Garne eine innere Komponente aus elastischen Fasern und eine äußere Komponente aus Natur- und/oder Kunstfasern umfassen.

9. Gewebe nach Anspruch 8, **dadurch gekennzeichnet, dass** die elastischen Fasern aus der Gruppe der Mikrofasern ausgewählt sind.

10. Gewebe nach Anspruch 9, **dadurch gekennzeichnet, dass** die Mikrofasern Polyamid- und/oder Polyurethanfasern sind.

11. Gewebe nach Anspruch 10, **dadurch gekennzeichnet, dass** die Mikrofasern aus Polyamid in einem Anteil von 70 % bis 95 %, vorzugsweise zwischen 75 % und 85 %, und Elastan in einem Anteil von 100 % bestehen.

12. Ein Funktionskleidungsstück, **dadurch gekennzeichnet, dass** es aus einem Gewebe nach einem der Ansprüche 1 bis 11 besteht, wobei die Membran auf der Seite des Gewebes angeordnet ist, die im Gebrauch der Haut des Trägers zugewandt ist.

13. Verfahren zur Herstellung von Geweben nach einem der Ansprüche 1 bis 11 zur Herstellung von Bandagen und/oder Kompressionsbekleidung, wobei das Verfahren folgende Schritte umfasst:
a) Bereitstellen einer elastokompressiven Schicht aus Garnen mit elastischer Komponente;
b) Aufbringen einer Mischung aus reinem medizinischem Silikonmaterial auf eine Seite der elastokompressiven Schicht;
c) Vernetzung der Mischung zur Gewinnung der Beschichtungsmembran;
wobei der Aufbringungsschritt b) durch Verteilen der Silikonmaterialmischung auf der elastokompressiven Schicht erfolgt.

## Revendications

1. Tissu destiné à la fabrication de bandages et/ou vêtements de compression, comprenant :
- une couche élastocompressive constituée de fils élastiques ;
- une membrane de revêtement perméable aux gaz appliquée sur une face de ladite couche élastocompressive ;
ladite membrane de revêtement étant une couche de gel de silicone pur de qualité médicale, obtenue par réticulation à partir d'un mélange initial de silicones purs de qualité médicale, comprenant un ou plusieurs élastomères, un ou plusieurs silicones à faible viscosité choisis parmi les cyclosilicones et un ou plusieurs silicones aux propriétés cicatrisantes ;
les élastomères étant présents dans ledit mélange initial à une proportion comprise entre 20 % et 40 % en poids, et le ou les silicones aux propriétés cicatrisantes étant présents dans ledit mélange initial à une proportion comprise entre 1 % et 10 % en poids;
**caractérisé en ce que** les silicones à faible viscosité choisis parmi les cyclosilicones sont présents à une proportion comprise entre 40 % et 70 % en poids.

2. Tissu selon la revendication 1, **caractérisé en ce que** ledit matériau silicone est de qualité médicale 1.

3. Tissu selon l'une quelconque des revendications précédentes, **caractérisé en ce que** ledit matériau silicone aux propriétés cicatrisantes est présent dans ledit mélange initial à une proportion proche de 5 % du poids dudit mélange.

4. Tissu selon l'une quelconque des revendications précédentes, **caractérisé en ce que** ledit mélange de matériaux silicones comprend du vinylpolysiloxane.

5. Tissu selon l'une quelconque des revendications précédentes, **caractérisé en ce que** ledit mélange de matériaux silicones est additionné d'un ou plusieurs principes actifs.

6. Tissu selon la revendication 5, **caractérisé en ce que** lesdits principes actifs sont choisis parmi les extraits végétaux, les complexes d'extraits végétaux, les complexes polyfonctionnels liposolubles et hydrosolubles.

7. Tissu selon l'une quelconque des revendications précédentes, **caractérisé en ce que** ledit gel de silicone est réparti par étalement à chaud sur une seule face de ladite couche élastocompressive.

8. Tissu selon l'une quelconque des revendications précédentes, **caractérisé en ce que** lesdits fils comprennent une composante interne en fibre élastique et une composante externe en fibre naturelle et/ou synthétique.

9. Tissu selon la revendication 8, **caractérisé en ce que** lesdites fibres élastiques sont choisies parmi les microfibres.

10. Tissu selon la revendication 9, **caractérisé en ce que** lesdites microfibres sont des fibres de polyamide et/ou de polyuréthane.

11. Tissu selon la revendication 10, **caractérisé en ce que** lesdites microfibres sont composées de polyamide en un pourcentage compris entre 70 % et 95 %, de préférence entre 75 % et 85 %, et d'élasthanne en un pourcentage complémentaire à 100 %.

12. Vêtement à membrane, **caractérisé en ce qu'**il est constitué d'un tissu selon l'une quelconque des revendications 1 à 11, dans lequel ladite membrane est répartie sur la face du tissu destinée à être en contact avec la peau de l'utilisateur.

13. Procédé de fabrication de tissus selon l'une quelconque des revendications 1 à 11 pour la production de bandages et/ou de vêtements de compression, comprenant les étapes suivantes :
a) fourniture d'une couche élastocompressive constituée de fils à composante élastique ;
b) application sur une face de ladite couche élastocompressive d'un mélange de silicones purs de qualité médicale ;
c) réticulation dudit mélange pour obtenir la membrane de revêtement ;
l'étape d'application b) étant réalisée par étalement dudit mélange de silicones sur ladite couche élastocompressive.
